# EUROPEAN PATENT SPECIFICATION

(11) **EP 2 749 285 B1**
(45) Date of publication and mention of the grant of the patent: **02.11.2016**
(21) Application number: 12829075.6
(22) Date of filing: 23.08.2012
(51) Int. Cl.: A61K 31/44, A61K 31/121, A61K 31/194, A61K 31/196, A61K 31/352, A61K 31/381, A61K 31/4035, A61K 31/4184, A61K 31/42, A61K 31/426, A61K 31/427, A61K 31/428, A61K 31/4439, A61K 38/00, A61K 45/00, A61P 19/08, A61P 43/00, A61K 45/06, A61K 38/13

(54) **OSTEOGENESIS PROMOTER AND USE THEREOF**
OSTEOGENESE-PROMOTOR UND SEINE VERWENDUNG
PROMOTEUR D'OSTÉOGENÈSE ET SON UTILISATION

(30) Priority: 26.08.2011 JP 2011185306
(43) Date of publication of application: 02.07.2014
(73) Proprietor: National University Corporation Nagoya University, Nagoya-shi, Aichi 464-8601 (JP)
(72) Inventor: OHNO, Kinji, Nagoya-shi Aichi 464-8601 (JP); ISHIGURO, Naoki, Nagoya-shi Aichi 464-8601 (JP); KITOH, Hiroshi, Nagoya-shi Aichi 464-8601 (JP); MISHIMA, Kenichi, Nagoya-shi Aichi 464-8601 (JP)
(74) Representative: Steinecke, Peter
(86) International application number: PCT/JP2012/071264
(87) International publication number: WO 2013/031620

(56) References cited:
- WO-A1-2008/016002
- US-A1- 2007 265 187
- US-A1- 2008 166 423
- SCOTT ROBERTS ET AL: "Functional Involvement of PHOSPHO1 in Matrix Vesicle-Mediated Skeletal Mineralization", JOURNAL OF BONE AND MINERAL RESEARCH, vol. 22, no. 4, 18 April 2007 (2007-04-18) , pages 617-627, XP55183537, ISSN: 0884-0431, DOI: 10.1359/jbmr.070108
- JOÃO COSTA-RODRIGUES ET AL: "Dose-dependent inhibitory effects of proton pump inhibitors on human osteoclastic and osteoblastic cell activity", FEBS JOURNAL, vol. 280, no. 20, 1 October 2013 (2013-10-01), pages 5052-5064, XP055183580, ISSN: 1742-464X, DOI: 10.1111/febs.12478
- CHUN, HJ. ET AL.: 'Effect of acid pump antagonist on differentiation and proliferation of osteoclasts/osteoblasts' JOURNAL OF GASTROENTEROLOGY AND HEPATOLOGY vol. 24, no. SUPP.1, 2009, page A16, XP008172866
- ZHAO, J. ET AL.: 'Icariin induces osteogenic differentiation in vitro in a BMP- and Runx2- dependent manner' BIOCHEM BIOPHYS RES COMMUN vol. 369, no. 2, 2008, pages 444 - 448, XP022551961
- YANG, L. ET AL.: 'Kaempferol Stimulates Bone Sialoprotein Gene Transcription and New Bone Formation' J CELL BIOCHEM vol. 110, no. 6, 2010, pages 1342 - 1355, XP055146034

## Description

### TECHNICAL FIELD

The present invention relates to a drug used for the formation or regeneration of bone tissues (osteogenesis promoter) and uses thereof. This application is based upon and claims the benefit of priority from prior Japanese Patent Application No. 2011-185306, filed August 26, 2011.

### BACKGROUND ART

Bone is one of tissues whose formation (regeneration) is expected to be achieved by regenerative medical techniques. Basic and applied researches aimed at the bone formation have been vigorously carried out, whereby various findings were made. For example, osteogenesis factor (bone morphogenetic protein: BMP) and retinoic acid are known as the substances which strongly induces differentiation from mesenchymal stem cells into the osteoblast lineage, and are widely used for in vitro researches. Of the clinically used drugs, those intensifies the action of BMP include vitamin D3, estrogen, and glucocorticoid. In addition, the substances which slowly induce the differentiation into the osteoblast lineage include the combination of dexamethasone, β-glycerophosphate, and ascorbic acid. The system containing them in a cell culture solution is frequently used in the study of in vitro differentiation.

The prior art references regarding osteogenesis are listed below. Patent document 1 relates a method for increasing the stability of Runx2, thereby activating the osteogenesis pathway by BMP. Patent document 2 suggests a method for promoting osteogenesis using the combination of a polyphosphonate, which is a main agent, with a proton pump inhibitor, thereby adjusting the intragastric pH, and supports the adsorption of the main agent.

### PRIOR ART DOCUMENT

### PATENT DOCUMENT

Patent Document 1: Japanese Unexamined Patent Application Publication No. 2008-523138
Patent Document 1: Japanese Unexamined Patent Application Publication No. 2001-253827

### SUMMARY OF THE INVENTION

### PROBLEM TO BE SOLVED BY THE INVENTION

BMP is not a cytokine which acts bone alone, and is a protein; so that its use is limited (oral administration is practically impossible). In addition, delivery of BMP to the region where bone neoplasticity is to be induced at the optimum concentration requires an adequate carrier. Furthermore, the production cost is high. Furthermore, BMP has a strong bone inducing ability, so it may cause bone neoplasticity (heterotopic ossification) in unintended regions. On the other hand, retinoic acid has teratogenicity, so its clinical use is markedly limited. In addition, vitamin D3, estrogen, and glucocorticoid are reported to have side effects such as promotion of bone absorption, hypercalcemia, and the development of ovarian cancer. The ex vivo induction of differentiation for bone marrow mesenchymal cells by the combination of dexamethasone, β-glycerophosphate, and ascorbic acid is applied to clinical researches, but it is not suitable for local and systemic administrations to the patient.

In view of the above-described circumstances, the present invention is intended to provide an osteogenesis promoter which can be administered locally or systemically, and is suitable for clinical application.

### MEANS FOR SOLVING PROBLEM

As a master transcription factor which induces the differentiation from mesenchymal stem cells to osteoblasts, Runx2 (runt-related transcription factor 2) is known. The expression of Runx2 is promoted by BMP-2. The present inventors focused attention on Runx2, and attempted to select low molecular weight compounds from existing drugs which specifically intensify the expression of Runx2. More specifically, firstly, the reporter vector prepared by linking 2 kb of the P1 promoter region of the normal human RUNX2 gene to the cDNA upstream of luciferase was transferred to the undifferentiated mouse mesenchymal cells line C3H10T1/2, thereby establishing a stable expression strain. The cells thus obtained were differentiated into osteoblasts by the addition of the BMP-2 to the culture solution, and further cultured for 24 hours in the presence of 1186 approved drugs (Prestwick Chemical), and subjected to screening by luciferase assay. In the primary screening, the cutoff value of promotion of the reporter activity was set at 1.5 times the vehicle ratio. 48 potential agents selected by the primary screening were subjected to the secondary screening under more strict conditions, and narrowed down to 15 potential agents. Of these, attention was given to nine agents having a track record in Japan, and concentration dependency of their activity was studied (tertiary screening). As a result of this, concentration dependency as to the promotion of *RUNX2* promoter activity was found in seven potential agents. Of the seven potential agents, five agents showed the action promoting *RUNX2* promoter activity. Of these five potential agents, two proton pump inhibitors (PPIs) which are widely used in clinical applications, and proved to be safe over a long term use were selected, and the gene expression of the endogenous *RUNX2* gene was studied by the real time PCR method using the BMP-2-induced C3H10T1/2 cell and human osteogenic sarcoma cell line (HOS); the concentration-dependent increase of the endogenous *RUNX2* gene was observed. These two approved drugs are lansoprazole (trade name TAKEPRON, Takeda Pharmaceutical Company Limited) and rabeprazole (trade name PARIET, Eisai Co., Ltd.) which are proton pump inhibitors (PPIs) used as drugs for peptic ulcer. As a further study, the Runx2 protein expression level and alkaline phosphatase (ALP) activity as the index of bone differentiation were evaluated over time using the BMP-2-induced C3H10T1/2 cells and the HOS cells by the Western blotting method and ELISA method, respectively. It was found that the expression level of the Runx2 protein increased in a concentration-dependent manner 48 hours and 72 hours after the addition of the PPI, and the alkaline phosphatase activity significantly increased on day 5 in the C3H10T1/2 cells and on day 6 in the HOS cells after the addition of the drug. More specifically, these two PPIs were found to have osteogenesis promoting effect. Also in the experiment using the rat bone marrow mesenchymal cells and human bone marrow mesenchymal stem cells (POIETICS (registered trademark), Lonza Ltd.), these PPIs were found to have osteogenesis promoting effect. On the other hand, in order to validate the in vivo effect of these PPIs, lansoprazole was applied to a rat femoral bone defect model, and good osteogenesis promoting effect was shown.

The following embodiments of the present invention are mainly based on the above-described results.
[1] An osteogenesis promoter which comprises one or more compounds selected from the group consisting of lansoprazole and rabeprazole, and their pharmaceutically acceptable salts for use in the therapeutic treatment of a bone fracture, a bone defect, or bone lengthening using distraction osteogenesis, the treatment involving the promotion of osteogenesis.
[2] The osteogenesis promoter for use according to claim 1, wherein the bone fracture includes traumatic bone fracture caused by a single impact and stress fracture caused by repeated loads.
[3] The osteogenesis promoter for use according to claim 1, wherein the bone defect includes traumatic bone defect, bone defect after removal of tumor, congenital pseudarthrosis, skeletal dysplasia, periodontal bone defect, and bone defect after correction of deformity.
[4] Use of one or more compounds selected from the group consisting of lansoprazole and rabeprazole, and their pharmaceutically acceptable salts for inducing differentiation from stem cells into osteoblasts in vitro.
[5] An in vitro method of providing osteoblasts suitable for use in transplantation to a patient the in vitro method comprising subjecting a culture solution of mesenchymal cells from the patient in vitro to one or more compounds selected from the group consisting of lansoprazole and rabeprazole, and their pharmaceutically acceptable salts and differentiation into osteoblasts.

### BRIEF DESCRIPTION OF THE DRAWINGS

Fig. 1 shows the expression inducing effect of PPI (rabeprazole or lansoprazole) on the *Runx2* gene. The HOS cells were used in the experiment. The abscissa is the concentration of PPI, and the ordinate is the relative expression level of the *Runx2* gene.
Fig. 2 shows the expression inducing effect of PPI (rabeprazole or lansoprazole) on the *Runx2* gene. The C3H10T1/2 cells differentiated by BMP-2 were used in the experiment. The abscissa is the concentration of PPI, and the ordinate is the relative expression level of the *Runx2* gene.
Fig. 3 shows the expression inducing effect of PPI (lansoprazole) on the Runx2 protein. The HOS cells were used in the experiment. The relative expression level to Gapdh (internal standard) was calculated.
Fig. 4 shows the expression inducing effect of PPI (rabeprazole) on the Runx2 protein. The HOS cells were used in the experiment. The relative expression level to Gapdh (internal standard) was calculated.
Fig. 5 shows the expression inducing effect of PPI (lansoprazole) on the Runx2 protein. The C3H10T1/2 cells differentiated by BMP-2 were used in the experiment. The relative expression level to Gapdh (internal standard) was calculated.
Fig. 6 shows the expression inducing effect of PPI (rabeprazole) on the Runx2 protein. The C3H10T1/2 cells differentiated by BMP-2 were used in the experiment. The relative expression level to Gapdh (internal standard) was calculated.
Fig. 7 shows the increase effect of PPI (rabeprazole or lansoprazole) on the ALP activity. The HOS cells were used in the experiment. The abscissa is the concentration of PPI, and the ordinate is the relative activity of ALP. *p < 0.05
Fig. 8 shows the increase effect of PPI (rabeprazole or lansoprazole) on the ALP activity. The C3H10T1/2 cells differentiated by BMP-2 were used in the experiment. The abscissa is the concentration of PPI, and the ordinate is the relative activity of ALP. *p < 0.05, **p < 0.1
Fig. 9 shows the expression inducing effect of PPI (rabeprazole or lansoprazole) on the *Runx2* gene. The rat bone marrow mesenchymal cells were used in the experiment. The abscissa is the concentration of PPI, and the ordinate is the relative expression level of *Runx2* gene. P1: first passage, P2: second passage, P3: third passage
Fig. 10 shows the expression inducing effect of PPI (rabeprazole or lansoprazole) on the *Runx2* gene. Human bone marrow mesenchymal cells were used in the experiment. The abscissa is the concentration of PPI, and the ordinate is the relative expression level of *Runx2* gene. P0: primary culture, P1: first passage, P2: second passage

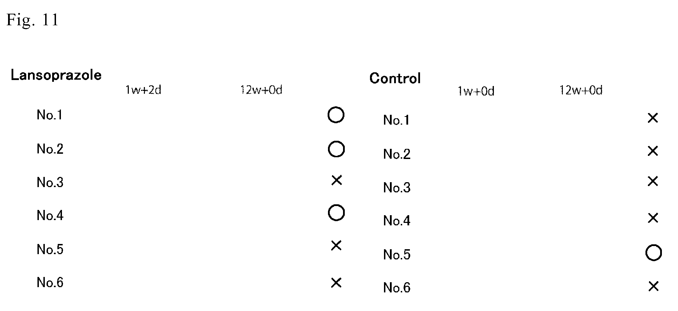

Fig. 11 shows the osteogenesis promoting effect of PPI (lansoprazole) in a rat femur defect model. Left: soft X-ray images of the bone defect parts in the groups No. 1 to 6 to which lansoprazole was administered. Right: soft X-ray images of the bone defect parts in the control groups No. 1 to 6. O: bone adhesion was found. X: bone adhesion was not found. For the treated groups, the photographs at the points of 1 week + 2 days (for No. 5, 0 week + 3 days) and 12 week + 0 day are shown. For the control groups, the photographs at the points of 1 week + 0 day (for No. 1, 1 week + 4 days) and 12 weeks + 0 day. For the treated group No. 6, the external fixator was loosened in 4 to 5 weeks, and osteomyelitis was found macroscopically. For the control groups No. 3, the external fixator was detached in 9 to 10 weeks.

### DESCRIPTION OF EMBODIMENTS for use as characterized in the claims

The present invention relates to an osteogenesis promoter (for convenience of explanation, the promoter is hereinafter referred to as "the drug of the present invention") for use as characterized in the claims. The "osteogenesis promoter" is a drug which acts on the stem cells or precursor cells having the potential ability to be differentiated into the osteoblast lineage, and induces differentiation into the osteoblast lineage, thereby promoting the formation of bone tissues. The drug of the present invention can be widely used for various diseases on which osteogenesis directly or indirectly has therapeutic effect. The drug of the present invention can be used in bone fracture, bone defect, or bone lengthening using distraction osteogenesis. The present drug is also useful in the in vitro induction of differentiation from stem cells into osteoblasts. Furthermore, systemic administration and local administration of the present drug to a region in need of osteogenesis allow the promotion of osteogenesis. The bone fracture includes traumatic bone fracture caused by a single impact, and stress fracture caused by repeated loads. The bone defect includes traumatic bone defect, bone defect after removal of tumor, congenital pseudoarthrosis, skeletal dysplasia, periodontal bone defect, and bone defect after correction of deformity.

The drug of the present invention includes one or more compounds selected from the group consisting of rabeprazole (2-[[4-(3-methoxypropoxy)-3-methylpyridin-2-yl]methylsulfinyl]-1H-benzimidazole (IUPAC name)), lansoprazole (2-[[3-methyl-4-(2,2,2-trifluoroethoxy)pyridin-2-yl]methylsulfinyl]-1H-benzimidazole (IUPAC name)), and their pharmaceutically acceptable salts as an active ingredient. These compounds were selected by the secondary screening based on the strict criteria (details are given in the below-described examples), and increase the promoter activity of the transcription factor Runx2. Accordingly, the drug of the present invention achieves therapeutic effects through the increase in the expression of Runx2. Runx2 is a master transcription factor inducing differentiation into osteoblasts. Runx2 is essential for osteoblast differentiation. Runx2 is known to act acceleratively in the early stage and suppressively in the later stage of osteoblast differentiation. The gene sequence and amino acid sequence of Runx2 are set forth in SEQ ID NO. 1 (DEFINITION: Homo sapiens runt-related transcription factor 2 (RUNX2), transcript variant 1, mRNA. ACCESSION: NM_001024630) and SEQ ID NO. 2 (DEFINITION: runt-related transcription factor 2 isoform a [Homo sapiens]. ACCESSION: NP_001019801), respectively.

The above-described compounds have track records of clinical application, and are readily available. For example, rabeprazole is commercially available as a proton pump inhibitor PARIET (trade name), and lansoprazole as a proton pump inhibitor TAKEPRON (trade name).

Rabeprazole, or lansoprazole is used as the active ingredient. These compounds were selected by the tertiary screening using the concentration dependency as the index. These compounds are also suitable for a long-term administration.

Rabeprazole, and lansoprazole were found to specifically promote the *RUNX2* promoter activity.

Rabeprazole, or lansoprazole is used as the active ingredient. These compounds are proton pump inhibitors (PPIs) which are widely used in clinical applications and proved to be safe over a long term of use. As a result of the study by the inventors, these two PPIs showed osteogenesis promoting effect in the experiment using culture cells. In addition, in vivo osteogenesis promoting effect was confirmed in the experiment using a model animal (lansoprazole was used as the test reagent).

In one embodiment of the present invention, based on the fact that osteogenesis promoting effect of the proton pump inhibitor (PPI) was observed, a PPI selected from rabeproazole, lansoprazole and their pharmaceutically acceptable salts is used. The benzimidazole PPI can be produced by a known method (for example, see Japanese Unexamined Patent Application Publication No. 52-62275, 54-141783, and 1-6270).

The active ingredient of the drug of the present invention may be a pharmaceutically acceptable salt of any of the above-described compounds. Examples of the "pharmaceutically acceptable salt" include acid-added salts, metal salts, ammonium salts, organic amine-added salts, and amino acid-added salts. Examples of the acid-added salts include inorganic acid salts such as hydrochlorides, sulfates, nitrates, phosphates, and hydrobromides, and organic acid salts such as acetates, maleates, fumarates, citrates, benzenesulfonates, benzoates, malates, oxalates, methanesulfonates, and tartrates. Examples of the metal salts include alkali metal salts such as sodium salts, potassium salts, and lithium salts, alkaline earth metal salts such as magnesium salts and calcium salts, aluminum salts, and zinc salts. Examples of the ammonium salts include the salts of ammonium and tetramethyl ammonium. Examples of the organic amine-added salts include morpholine-added salts and piperidine-added salts. Examples of the amino acid-added salts include glycine-added salts, phenylalanine-added salts, lysine-added salts, aspartic acid-added salts, and glutamic acid-added salts.

An optical isomer of any of the above-described compounds may be used as long as it exhibits desired activity. When an optical isomer is present, it may be used as the active ingredient in the form of a racemic body or a substantially pure enantiomer.

The formulation of the drug of the present invention may use a common procedure. When formulated, other components which are acceptable for formulation (for example, a carrier, an excipient, a disintegrating agent, a buffering agent, an emulsifying agent, a suspending agent, a soothing agent, a stabilizer, a preservative, an antiseptic, and a normal saline solution) may be added. Examples of the excipient include lactose, starch, sorbitol, D-mannitol, and white sugar. Examples of the disintegrating agent include starch, carboxymethyl cellulose, and calcium carbonate. Examples of the buffering agent include phosphates, citrates, and acetates. Examples of the emulsifying agent include gum arabic, sodium alginate, and tragacanth. Examples of the suspending agent include glycerol monostearate, monostearic acid aluminum, methyl cellulose, carboxymethyl cellulose, hydroxymethyl cellulose, and sodium lauryl sulfate. Examples of the soothing agent include benzyl alcohol, chlorobutanol, and sorbitol. Examples of the stabilizer include propylene glycol, and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

The form of formulation is not particularly limited. Examples of the form include pellets, a powder, fine pellets, granules, capsules, a syrup, an injection, an external preparation, and a suppository. The drug of the present invention is administered to the subject orally or parenterally (for example, intravenous, intraarterial, hypodermic, intradermal, intramuscular, or intraperitoneal injection, transdermal, nasotracheal, or transmucosal administration). Systemic and local administrations are appropriately used according to the subject. These administration routes are not exclusive each other, and optionally selected two or more may be combined (for example, intravenous injection or the like is carried out concurrently with oral administration or after a lapse of a predetermined period from finishing of oral administration). The drug of the present invention contains an active ingredient in an amount necessary for achieving the expected therapeutic effect (more specifically a therapeutically effective amount). The amount of the active ingredient in the drug of the present invention generally depends on the drug shape. In order to achieve the intended dose, the amount of the active ingredient is, for example, about 0.1% to 99% by weight.

Also disclosed is an osteogenesis method using the drug of the present invention. The osteogenesis includes administering the drug of the present invention to a patient in need of osteogenesis. Examples of the clinical diseases which require osteogenesis include bone fracture, bone defect, and bone lengthening utilizing distraction osteogenesis. Bone fractures include traumatic bone fracture caused by a single impact, and stress fracture caused by repeated loads. The bone defect includes traumatic bone defect, bone defect after removal of tumor, congenial pseudoarthrosis, skeletal dysplasia, periodontal bone defect, and bone defect after correction of deformity of distal radius fracture and spinal fracture (compression fracture) associated with osteoporosis.

The dose of the drug of the present invention is established to achieve the expected therapeutic effects. For the establishment of the therapeutically effective dose, in general, the symptoms, the age, sex, and body weight of the patient, and other factors are taken into consideration. Those skilled in the art can establish an appropriate dose in consideration of these factors. For example, the dose for an adult (body weight: about 60 kg) may be established in such a manner that the amount of the active ingredient is about 0.1 mg to 1,000 mg a day. The administration schedule may be, for example, once to several times a day, once every two days, or once every three days. For the making of the administration schedule, the disease state of the patient, and the expected duration of the effect of the active ingredient may be taken into consideration. Examples of the method of local administration include the use during surgery, and injection into the intended location in the form of a carrier or any appropriate shape for the purpose of promoting the hearing process.

The treatment using the drug of the present invention may be carried out in parallel with treatment using other drug (for example, an existing therapeutic drug). Alternatively, an existing therapeutic procedure may be combined with the treatment using the drug of the present invention. Examples of the existing therapeutic procedures include bone lengthening. Bone lengthening uses a fixing device (internal or external fixing type) or a special device called, for example, a bone lengthener. The method of bone lengthening usually includes the processes of osteotomy, waiting period, bone lengthening period, and bone consolidation period. When the drug of the present invention is used in combination with bone lengthening, usually, the drug of the present invention is locally administered during the period from the starting of the waiting period to finishing of the bone consolidation period. The frequency of administration is not particularly limited. For example, the drug is administered once to ten times. Details about bone lengthening are described in, for example, ADVANCE SERIES II-9, Hone Enchojutsu Saikin No Shinpo (Bone Lengthening: Recent advance (Kokuseido Co., Ltd., supervised by Kiyonori Harii, edited by Tsuneki Sugihara).

### Examples

### <Screening of compounds having osteogenesis promoting effect>

In order to find the compound having osteogenesis promoting effect, the low molecular weight compounds which specifically increase the expression of the transcription factor Runx2 were selected from the approved drugs.

### 1. Primary screening

### (1) Method

Existing drugs which increase the promoter activity of the transcription factor Runx2 were exhaustively searched by luciferase assay. Firstly, the P1 promoter region of the human Runx2 gene was inserted into the multiple cloning site of the pGL4 luciferase reporter vector (Promega K.K.) (pGL4.10 vector). The pGL4.10 vector was transfected into the undifferentiated mouse mesenchymal cell line C3H10T1/2, and selectively cultured on a G418-containing medium, thereby establishing the stable expression strain. The cells were seeded on a 96-well (day 1), and human BMP-2 was added to each well on day 2, thereby inducing differentiation into osteoblasts. On the following day, the test compounds (10 µM) composing Prestwick Chemical Library (registered trademark) (Prestwick Chemical Ltd.) were added, and the cells were collected 24 hours after, and subjected to luciferase assay. The luciferase assay was repeated three times, and the compounds which made the reporter activity (luminescence) of luciferase 1.5 times or higher that of the control (vehicle) even at once were selected.

### (2) Result

As a result of screening based on the above-described criterion, 48 potential agents were selected.

### 2. Secondary screening

### (1) Method

In order to exclude the influence of the variations in sensitivity of the measurement instrument and difference in pipetting, the 48 potential agents selected by the primary screening were further subjected to total five times of (N = 6/time) screening. In addition to the compounds which made the reporter activity of luciferase (average of 5 times) 1.5 times that of the control, those made the activity 1.5 times than that of the control even at once were selected.

### (2) Result

As a result of screening based on the above-described criterion, 15 potential agents (phenazopyridine hydrochloride, riluzole hydrochloride, tranilast, rabeprazole, indoprofen, nabumetone, luteolin, leflunomide, lansoprazole, methiazole, tiabendazole, albendazole, tiaprofenic acid, balsalazide sodium, and cyclosporin A) were selected.

### 3. Tertiary screening

### (1) Method

Of the 15 potential agents selected by the secondary screening, 9 agents practically used in Japan (riluzole hydrochloride, tranilast, rabeprazole, nabumetone, leflunomide, lansoprazole, albendazole, tiaprofenic acid, and cyclosporin A) were subjected to total three times of screening (N = 6/time) at 8 levels of concentration, thereby determining whether the reporter-activity has a dependence on the concentration or not.

### (2) Result

Seven potential agents (riluzole hydrochloride, tranilast, rabeprazole, nabumetone, leflunomide, lansoprazole and tiaprofenic acid) showed a concentration dependency of the promotion of RUNX2 promoter activity. In order to exclude the possibility of the potential agent to increase the enzymatic activity of luciferase protein, the pGL4.10 basic vector (pGL4.10 [luc2/SV40] basic vector) and phRLvector (phRL [hRluc/TK] vector) were cotransfected into the C3H10T1/2 cells, the potential agents were individually added, and double luciferase assay was performed. As a result of this, five potential agents (rabeprazole, nabumetone, leflunomide, lansoprazole and tiaprofenic acid) were found to specifically promote the RUNX2 promoter activity. The other two potential agents (riluzole hydrochloride and tranilast) were found to slightly increase the luciferase activity, so that were removed from the subject of further study. Of these five potential agents, two proton pump inhibitors (PPIs) which are widely used in clinical applications and proved to be safety over a long term of use (rabeprazole and lansoprazole) were decided as the final potential agent, and their osteogenesis promoting action was studied.

### 4. Osteogenesis promoting effect by selected PPIs

### 4-1. Induction of development of Runx2 gene (study on cell line)

### (1) Method

Human osteogenic sarcoma cells (HOSs) and the C3H10T1/2 cells were used in the experiment. Firstly, the cells (HOS or C3H10T1/2) were seeded on a 12-well plate (day 1). When the C3H10T1/2 cells were used, the human BMP-2 was added to each well on the following day. On day 3, PPI (rabeprazole or lansoprazole) was added and cultured for 24 hours, and the cells were collected. Total RNAs were prepared from the collected cells, and the expression of the Runx2 gene was analyzed by the quantitative PCR method.

### (2) Result

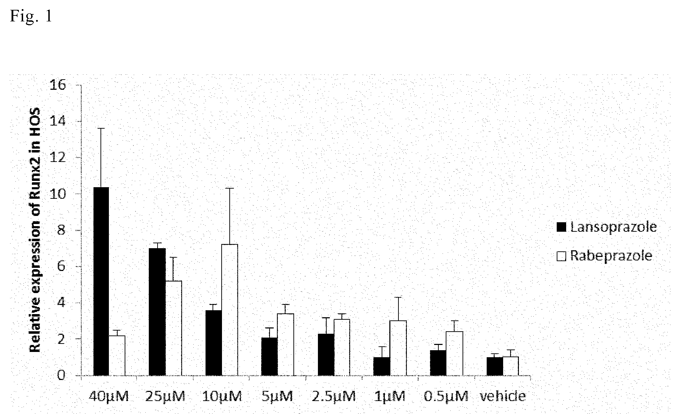

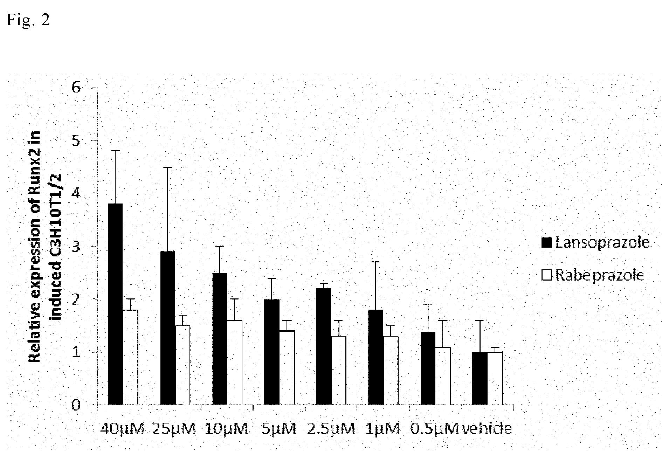

Both of rabeprazole and lansoprazole showed concentration-dependent activity of inducing the expression of the Runx2 gene (Figs. 1 and 2) .

### 4-2. Induction of expression of Runx2 protein and increase of alkaline phosphatase (ALP) activity (study on cell line)

### (1) Method

Using the HOS cells and the C3H10T1/2 cells cultured in the same manner as in 4-1, and the expression level of the Runx2 protein and the ALP activity which is the index of bone differentiation were evaluated over time. The expression level of the Runx2 protein was evaluated by the Western blot test, and the ALP activity was measured by the ELISA method.

### (2) Result

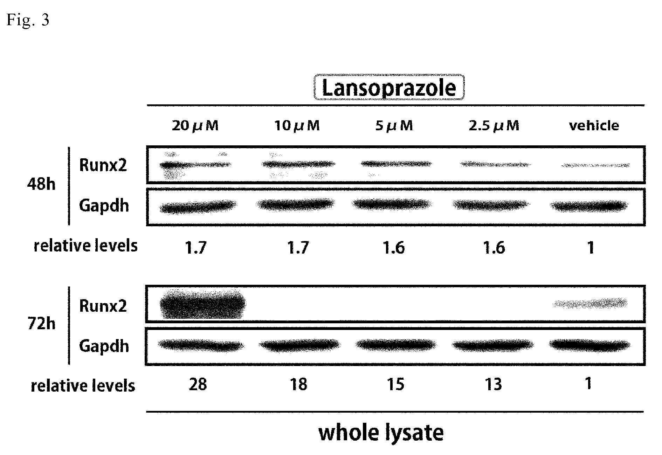

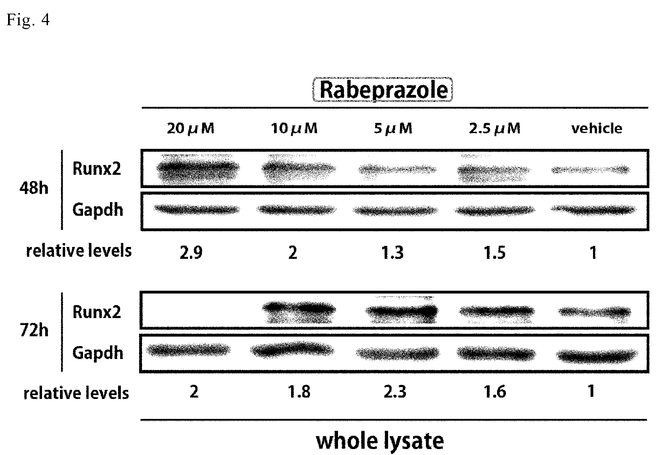

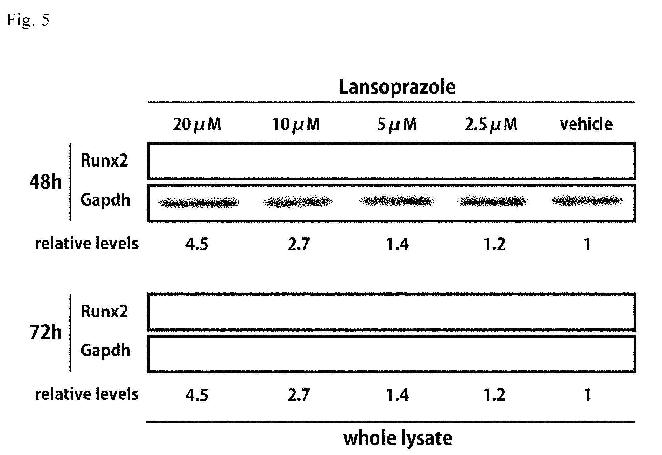

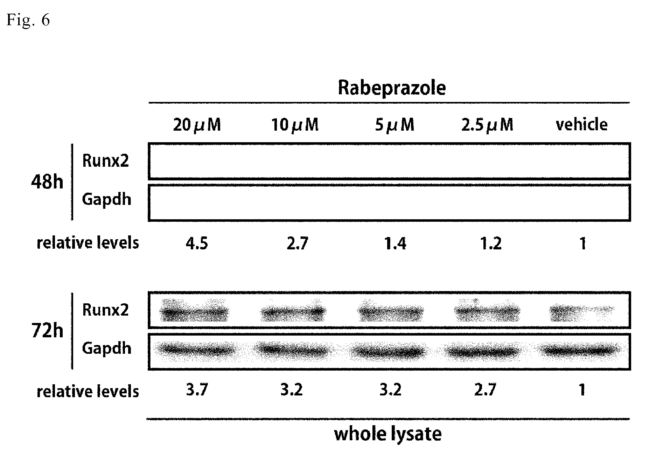

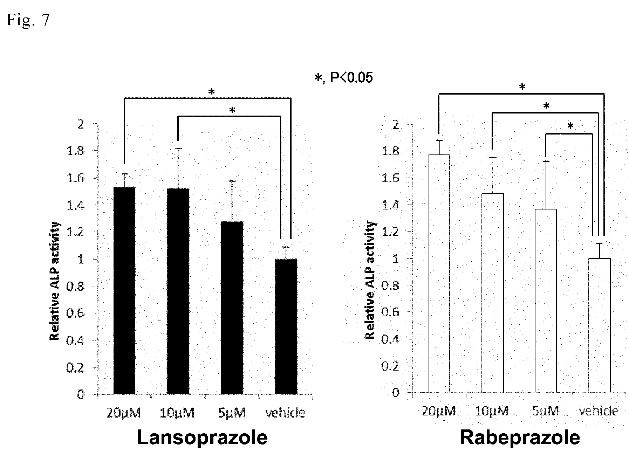

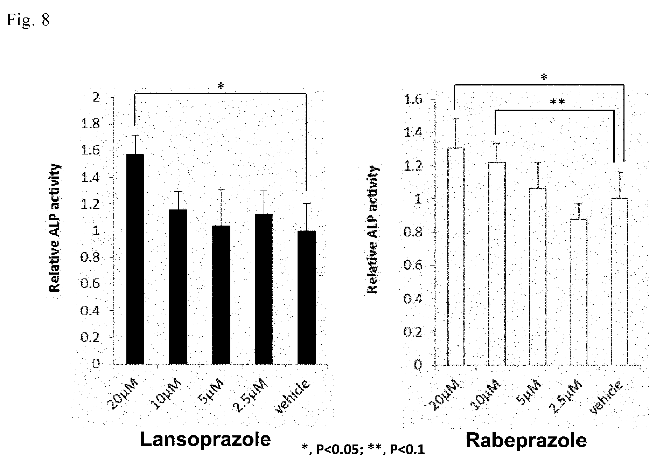

48 hours and 78 hours after the addition of PPI, the concentration-dependent increase in the Runx2 protein expression level was found (FIGs. 3 to 6). The ALP activity significantly increased on day 6 (for the HOS cells; see Fig. 7) and day 5 (for the BMP-2-induced C3H10T1/2 cells; see Fig. 8) after the addition of PPI.

### 4-3. Induction of Expression of Runx2 gene (study on bone marrow cells)

### (1) Method

Mesenchymal cells were prepared from a rat bone marrow solution, and cultured (cultured bone marrow mesenchymal cells). Subsequently, the cells were subcultivated (P1, P2, and P3) in bone-inducing media (dexamethasone, β-glycerophosphoric acid, and ascorbic acid were added the culture media). PPI was added when each of the P1, P2, and P3 reached confluent. The cells were collected 24 hours after the addition of PPI, and the expression of the Runx2 gene was analyzed in the same manner as in 4-1.

On the other hand, the human bone marrow mesenchymal stem cells (POIETICS (registered trademark), Lonza Ltd.) were subjected to primary culture (P0) and subcultivation (P1, P2) in the PPI-containing bone-inducing media (culture media containing PPI, dexamethasone, β-glycerophosphoric acid, and ascorbic acid). The bone-inducing medium was replaced every three days. The cells were collected when each of P0, P1, and P2 reached confluent, and the expression of the Runx2 gene was analyzed in the same manner as in 4-1.

### (2) Result

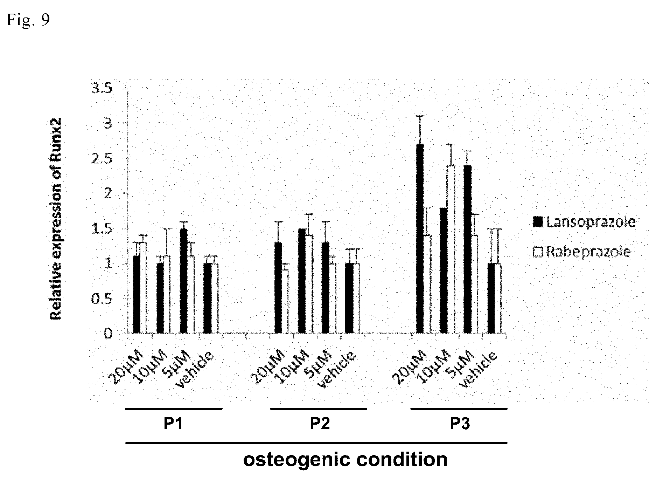

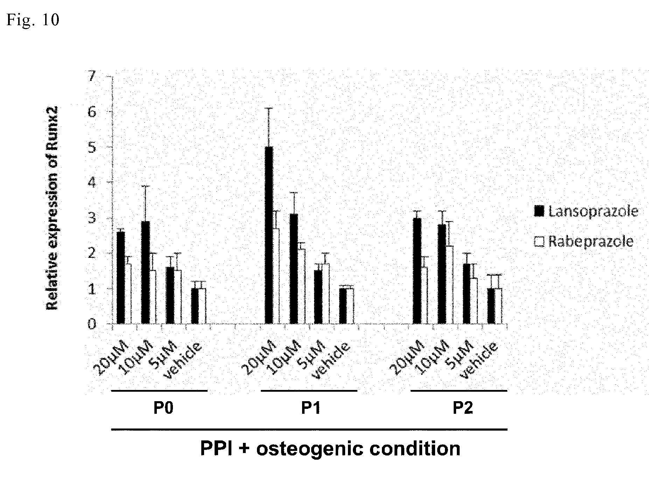

In the case of rat cultured bone marrow cells, PPI concentration-dependent induction of the expression of the Runx2 gene was observed in P3 (Fig. 9). On the other hand, for the human bone marrow mesenchymal stem cells, PPI concentration-dependent induction of the expression of the Runx2 gene was observed in the early stage, and the effect lasted (Fig. 10).

### 4-4. Osteogenesis promoting effect in rat femur defect model

### (1) Method

The left hind femur of an SD rat (male, 9 weeks old) was exposed by anterolateral approach, and an external fixator was installed. The diaphysis was cut by an oscillating saw, (defect amount 3 mm), and the bone was elongated in a single stage, and used as a bone defect model (defect group N = 6, control group N = 6). Lansoprazole was given to the bone defect model by *ad libitum* oral administration (about 10 to 20 times the usual human dose), and the bone defect part was photographed periodically by soft X-ray. In addition, the body weight and drinking water amount were periodically measured.

### (2) Result

The group treated with lansoprazole showed good osteogenesis in half of them (3/6) (Fig. 11, left). This result indicates that lansoprazole exhibits marked osteogenesis promoting effect.

### INDUSTRIAL APPLICABILITY

The osteogenesis promoter of the present invention promotes osteogenesis through the promotion of the expression of Runx2. For example, the application of the present invention to bone fracture, bone defect, or bone lengthening using distraction osteogenes is expected. Bone fracture includes traumatic bone fracture caused by a single impact, and stress fracture caused by repeated loads. Bone defect includes traumatic bone defect, bone defect after removal of tumor, congenial pseudoarthrosis, skeletal dysplasia, and periodontal bone. Ex vivo treatment is possible, which is carried out by, for example, the osteogenesis promoter of the present invention is acted in vitro on the mesenchymal cells collected from the bone marrow of the patient (for example, the osteogenesis promoter of the present invention is added to the culture solution), the differentiation into osteoblasts is promoted, and then transplanted into the patient (for example, returned to the long bone).

The use of an approved drug as the active ingredient of the present invention is advantageous in clinical application. More specifically, when an approved drug is used as the active ingredient, establishment of the usage and dose is relatively easy. Lansoprazole and rabeprazole are widely used as a drug for peptic ulcer in clinical applications, and are proved to be safe regarding the optimal dose, side effect, and contraindication.

## Claims

1. An osteogenesis promoter which comprises one or more compounds selected from the group consisting of lansoprazole and rabeprazole, and their pharmaceutically acceptable salts for use in the therapeutic treatment of a bone fracture, a bone defect, or bone lengthening using distraction osteogenesis, the treatment involving the promotion of osteogenesis.

2. The osteogenesis promoter for use according to claim 1, wherein the bone fracture includes traumatic bone fracture caused by a single impact and stress fracture caused by repeated loads.

3. The osteogenesis promoter for use according to claim 1, wherein the bone defect includes traumatic bone defect, bone defect after removal of tumor, congenital pseudarthrosis, skeletal dysplasia, periodontal bone defect, and bone defect after correction of deformity.

4. Use of one or more compounds selected from the group consisting of lansoprazole and rabeprazole, and their pharmaceutically acceptable salts for inducing differentiation from stem cells into osteoblasts *in vitro.*

5. An *in vitro* method of providing osteoblasts suitable for use in transplantation to a patient the in vitro method comprising subjecting a culture solution of mesenchymal cells from the patient *in vitro* to one or more compounds selected from the group consisting of lansoprazole and rabeprazole, and their pharmaceutically acceptable salts and differentiation into osteoblasts.

## Patentansprüche

1. Osteogenese-Promotor, welcher eine oder mehrere Verbindungen ausgewählt aus der Gruppe bestehend aus Lansoprazol und Rabeprazol umfasst, und deren pharmazeutisch verträgliche Salze zur Verwendung in einer therapeutischen Behandlung einer Knochenfraktur, eines Knochendefekts oder einer Knochenverlängerung unter Einsatz von Distraktionsosteogenese, wobei die Behandlung Unterstützung von Osteogenese involviert.

2. Osteogenese-Promotor zur Verwendung gemäß Anspruch 1, wobei die Knochenfraktur eine traumatische Knochenfraktur, verursacht durch einen einzelnen Aufprall und Stressfraktur verursacht durch wiederholte Belastung einschließt.

3. Osteogenese-Promotor zur Verwendung gemäß Anspruch 1, wobei der Knochendefekt einen traumatischen Knochendefekt, Knochendefekt nach Entfernung eines Tumors, kongenitale Pseudarthrose, Skelettdysplasie, periodontalen Knochendefekt und Knochendefekt nach Korrektur von Deformation einschließt.

4. Verwendung von einem oder mehreren Verbindungen ausgewählt aus der Gruppe bestehend aus Lansoprazol und Rabeprazol und deren pharmazeutisch verträglichen Salzen zur Induktion von Differenzierung von Stammzellen zu Osteoblasten *in vitro.*

5. *In vitro* Verfahren zur Bereitstellung von Osteoblasten geeignet zur Verwendung bei Transplantation an einen Patienten, das *in vitro* Verfahren umfassend Aussetzen einer Kulturlösung von mesenchymalen Zellen aus dem Patienten *in vitro* einer oder mehreren Verbindungen ausgewählt aus der Gruppe bestehend aus Lansoprazol und Rabeprazol und deren pharmazeutisch verträglichen Salzen und Differenzierung zu Osteoblasten.

## Revendications

1. Promoteur de l'ostéogenèse qui comprend un ou plusieurs composés choisis dans le groupe constitué de lansoprazole et de rabéprazole, et de leurs sels pharmaceutiquement acceptables pour une utilisation dans le traitement thérapeutique d'une fracture osseuse, d'un défaut osseux ou d'un allongement osseux utilisant une distraction osseuse, le traitement impliquant la promotion de l'ostéogenèse.

2. Promoteur de l'ostéogenèse pour son utilisation selon la revendication 1, dans lequel la fracture osseuse comporte une fracture osseuse traumatique provoquée par un choc unique et une fracture de fatigue provoquée par des charges répétées.

3. Promoteur de l'ostéogenèse pour une utilisation selon la revendication 1, dans lequel le défaut osseux comporte un défaut osseux traumatique, un défaut osseux après l'excision d'une tumeur, la pseudarthrose congénitale, la dysplasie squelettique, un défaut osseux parodontal et un défaut osseux consécutif à la correction d'une malformation.

4. Utilisation d'un ou plusieurs composés choisis dans le groupe constitué de lansoprazole et de rabéprazole, et de leurs sels pharmaceutiquement acceptables pour induire une différenciation des cellules souches en ostéoblastes in vitro.

5. Procédé in vitro de fourniture d'ostéoblastes appropriés pour une utilisation dans une greffe à un patient, le procédé in vitro comprenant la soumission d'une solution de culture de cellules mésenchymateuses du patient in vitro à un ou plusieurs composés choisis dans le groupe constitué de lansoprazole et de rabéprazole, et de leurs sels pharmaceutiquement acceptables et la différenciation en ostéoblastes.


### SEQUENCE LISTING

<110> NATIONAL UNIVERSITY CORPORATION NAGOYA UNIVERSITY
   OHNO, Kinji
   ISHIGURO, Naoki
   KITOH, Hiroshi
   MISHIMA, Kenichi
<120> Osteogenesis promoter and use thereof
<130> NA31A06/P-EP/WO (NU11005PE)
<150> JP P2011-185306
   <151> 2011-08-26
<160> 2
<170> Patent In version 3.5
<210> 1
   <211> 5553
   <212> DNA
   <213> Homo sapiens
<400> 1
   gtgtgaatgc ttcattcgcc tcacaaacaa ccacagaacc acaagtgcgg tgcaaacttt      60
   ctccaggagg acagcaagaa gtctctggtt tttaaatggt taatctccgc aggtcactac     120
   cagccaccga gaccaacaga gtcatttaag gctgcaagca gtatttacaa cagagggtac     180
   aagttctatc tgaaaaaaaa aggagggact atggcatcaa acagcctctt cagcacagtg     240
   acaccatgtc agcaaaactt cttttgggat ccgagcacca gccggcgctt cagccccccc     300
   tccagcagcc tgcagcccgg caaaatgagc gacgtgagcc cggtggtggc tgcgcaacag     360
   cagcagcaac agcagcagca gcaacagcag cagcagcagc agcaacagca gcagcagcag     420
   caggaggcgg cggcggcggc tgcggcggcg gcggcggctg cggcggcggc agctgcagtg     480
   ccccggttgc ggccgcccca cgacaaccgc accatggtgg agatcatcgc cgaccacccg     540
   gccgaactcg tccgcaccga cagccccaac ttcctgtgct cggtgctgcc ctcgcactgg     600
   cgctgcaaca agaccctgcc cgtggccttc aaggtggtag ccctcggaga ggtaccagat     660
   gggactgtgg ttactgtcat ggcgggtaac gatgaaaatt attctgctga gctccggaat     720
   gcctctgctg ttatgaaaaa ccaagtagca aggttcaacg atctgagatt tgtgggccgg     780
   agtggacgag gcaagagttt caccttgacc ataaccgtct tcacaaatcc tccccaagta     840
   gctacctatc acagagcaat taaagttaca gtagatggac ctcgggaacc cagaaggcac     900
   agacagaagc ttgatgactc taaacctagt ttgttctctg accgcctcag tgatttaggg     960
   cgcattcctc atcccagtat gagagtaggt gtcccgcctc agaacccacg gccctccctg    1020
   aactctgcac caagtccttt taatccacaa ggacagagtc agattacaga ccccaggcag    1080
   gcacagtctt ccccgccgtg gtcctatgac cagtcttacc cctcctacct gagccagatg    1140
   acgtccccgt ccatccactc taccaccccg ctgtcttcca cacggggcac tgggcttcct 1200
   gccatcaccg atgtgcctag gcgcatttca gatgatgaca ctgccacctc tgacttctgc 1260
   ctctggcctt ccactctcag taagaagagc caggcaggtg cttcagaact gggccctttt 1320
   tcagacccca ggcagttccc aagcatttca tccctcactg agagccgctt ctccaaccca 1380
   cgaatgcact atccagccac ctttacttac accccgccag tcacctcagg catgtccctc 1440
   ggtatgtccg ccaccactca ctaccacacc tacctgccac caccctaccc cggctcttcc 1500
   caaagccaga gtggaccctt ccagaccagc agcactccat atctctacta tggcacttcg 1560
   tcaggatcct atcagtttcc catggtgccg gggggagacc ggtctccttc cagaatgctt 1620
   ccgccatgca ccaccacctc gaatggcagc acgctattaa atccaaattt gcctaaccag 1680
   aatgatggtg ttgacgctga tggaagccac agcagttccc caactgtttt gaattctagt 1740
   ggcagaatgg atgaatctgt ttggcgacca tattgaaatt cctcagcagt ggcccagtgg 1800
   tatctggggg ccacatccca cacgtatcaa tatatacata tatagagaga gtgcatatat 1860
   atgtatatcg attagctatc tacaaagtgc ctatttttta gaagattttt cattcactca 1920
   ctcagtcatg atcttgcagc cataagaggg tagatattga gaagcagaag gctcaagaga 1980
   gacaattgca atcgagcttc agattgttta ctatttaaga tgtactttta caaaggaaca 2040
   aagaagggaa aaggtatttt tgtttttgtt gtttggtctg ttatcatcaa taacctgttc 2100
   atatgccaat tcagagaggt ggactccagg ttcaggaggg agaagagcaa agccgcttcc 2160
   tctctgtgct ttgaaacttc acaccctcac ggtggcagct gtgtatggac cagtgccctc 2220
   cgcagacagc tcacaaaacc agttgaggtg cactaaaggg acatgaggta gaatggatgc 2280
   ttccatcaca gtaccatcat tcagaataac tcttccaatt tctgctttca gacatgctgc 2340
   aggtcctcat ctgaactgtt gggttcgttt tttttttttt ttttcctgct ccaagaaagt 2400
   gacttcaaaa ataactgatc aggatagatt attttatttt actttttaac actccttctc 2460
   ccctttcccc actgaaccaa aaagaaatcc catccctaaa acctgccttc tccttttatg 2520
   caaaactgaa aatggcaata cattattata gccataatgg tatagatagt gattgcgttt 2580
   ggctatgtgt tgttttcttt ttttttaaat tatgaatatg tgtaaaatct gaggtaactt 2640
   gctaacgtga atggtcatat aactttaaag atatatttat aattatttaa tgacatttgg 2700
   acccttgaaa catttcttag tgtattgata tgttgacttc ggtctctaaa agtgctcttt 2760
   attaaataac aaatttcttc agtggtctag agccatatct gaaatattgc taagcaattt 2820
   cagttcatcc aggcacaatg tgattttaaa aaatacttcc atctccaaat attttagata 2880
   tagattgttt ttgtgatgta tgaaggaaat gttatgttta gttctttcag atctttgaat 2940
   gcctctaaca cagctttgcc ttctaaagcg gtaattaggg atttaaaaaa caacctttag 3000
   ccctttatca gcatgaaatg ctggagtgat gtggttttct aatttctttg gggtaattat 3060
   gactcttgtc atattaaaaa gacaagcaca agtaaatcat tgaactacag aaaaatgttc 3120
   tgtggtttca tagttaagca aaactctaaa tcgccaggct tcatagcaaa gacatagtca 3180
   gctaaaagcc gcacatgtgg atagagggtt caattatgag acacctagta caggagagca 3240
   aaattgcacc agagattctt aaccaaccag ccttaccaaa caacacaaca ggggaacccc 3300
   aatctgcctt acccaaggcc ccactggcag ctttccacag aatttgcatt tagaggagca 3360
   gaatgacatc actgtccttt gggagtaggt cctctgaaaa ggcagcaggt tccagcaggt 3420
   agctgagctg agaggacata tggcccacgg ggacctacag acagcctttg acatttgtat 3480
   ttcttacaat ggagggccaa ggagggcaag gggctgtgga gtttggtgtc tactagtgtg 3540
   tatgaatttg agctagagtc cttctgtggc atgcactttg accactcctg gcagtcacat 3600
   ggcagatttc caagtgcaaa tccttaatcc aaacaaggat catctaatga caccaccagg 3660
   ccaatccctg ctctcctccc cgaaaagtca gggtcccttc attggaatcc tccacccacc 3720
   caagcagaat ttagcagaga tttgccttca aaccctaacg gcccccttgt tctctggtcc 3780
   ttctcaaacc cacctttgta ggccacccag cattgcagga cagcgtgtgg ggcagctgga 3840
   cctgtgcttc ctgcctggga gtctcccttg gaattcatcc tgactccttc taataaaaat 3900
   ggatgggaaa gcaaaacact ttgccttcta aaggccgtat accaagtatg cttagataaa 3960
   taagccactt ttctattact taagtaagaa ggaagtagta attgatacta tttattgttt 4020
   gtgtgtggta gcttgaagca caccactgtc catttatttg taagtgtaaa atatgtgtgt 4080
   ttgtttcagc agcacttaaa aaagccagtg tctggttaca catttcaatt ttaattaatt 4140
   gacataaaaa tgctaccgcc agtgccagct gcatcctatt taattaaaaa ggtactatat 4200
   ttgtacatta ttttttaatg ttaaaagggc ttttttaagt ttacagtaca cataccgagt 4260
   gactttaggg atgcttttgt gttgaaatgt tactatagtg gctgcaggca gcaacccaga 4320
   aacactttag aagctttttt tccttgggaa aaattcaagc acttcttccc tccaccctca 4380
   ctccaaccac cccaatgggg gtaattcaca tttcttagaa caaattctgc ccttttttgg 4440
   tctagggatt aaaattttgt ttttctttct ttcttttttt ttttttttca ctgaaccctt 4500
   aatttgcact gggtcatgtg tttgatttgt gatttcaaga ccaaagcaaa gtcttactac 4560
   tactgtggaa ccatgtacta gttcctggga attaaaatag cgtggttctc tttgtagcac 4620
   aaacattgct ggaatttgca gtcttttcaa tgcagccaca tttttatcca tttcagttgt 4680
   ctcacaaatt ttaacccata tcagagttcc agaacaggta ccacagcttt ggttttagat 4740
   tagtggaata acattcagcc cagaactgag aaactcaaca gattaactat cgtttgctct 4800
   ttagacggtc tcactgcctc tcacttgcca gagccctttc aaaatgagca gagaagtcca 4860
   caccattagg gaccatctgt gataaattca gaagggagga gatgtgtgta cagctttaag 4920
   gattccctca attccgagga aagggactgg cccagaatcc aggttaatac atggaaacac      4980
   gaagcattag caaaagtaat aattatacct atggtatttg aaagaacaat aataaaagac      5040
   acttcttcca aaccttgaat ttgttgtttt tagaaaacga atgcatttaa aaatattttc      5100
   tatgtgagaa ttttttagat gtgtgtttac ttcatgttta caaataactg tttgcttttt      5160
   aatgcagtac tttgaaatat atcagccaaa accataactt acaataattt cttaggtatt      5220
   ctgaataaaa ttccatttct tttggatatg ctttaccatt cttaggtttc tgtggaacaa      5280
   aaatatttgt agcattttgt gtaaatacaa gctttcattt ttattttttc caattgctat      5340
   tgcccaagaa ttgctttcca tgcacatatt gtaaaaattc cgctttgtgc cacaggtcat      5400
   gattgtggat gagtttactc ttaacttcaa agggactatt tgtattgtat gttgcaactg      5460
   taaattgaat tatttggcat ttttctcatg attgtaatat taatttgaag tttgaattta      5520
   attttcaata aaatggcttt tttggttttg tta                                   5553
<210> 2
   <211> 521
   <212> PRT
   <213> Homo sapiens
<400> 2